# NEW EUROPEAN PATENT SPECIFICATION

(11) **EP 2 688 427 B2**
(45) Date of publication and mention of the opposition decision: **18.05.2022**
(45) Mention of the grant of the patent: 29.07.2015
(21) Application number: 12719405.8
(22) Date of filing: 26.03.2012
(51) Int. Cl.: A23C 11/10, A23L 2/52, A61K 31/575, A23L 33/11

(54) **SERUM CHOLESTEROL LOWERING DRINK**
SERUM-CHOLESTERIN-SENKENDES GETRÄNK
BOISSON A EFFET REDUCTEUR DU CHOLESTEROL SANGUIN

(30) Priority: 25.03.2011 FI 20110110; 25.03.2011 US 201161467757 P
(43) Date of publication of application: 29.01.2014
(73) Proprietor: Raisio Nutrition Ltd, 21200 Raisio (FI)
(72) Inventor: KUUSISTO, Päivi, FI-21200 Raisio (FI); WESTER, Ingmar, FI-21200 Raisio (FI)
(74) Representative: Carpmaels & Ransford LLP
(86) International application number: PCT/FI2012/000018
(87) International publication number: WO 2012/131145

(56) References cited:
- EP-A1- 0 911 385
- WO-A1-2004/093571
- WO-A1-2005/013707
- WO-A1-2009/068651
- WO-A1-2009/071737
- WO-A1-2010/084240
- WO-A2-00/41491
- WO-A2-2009/013395
- DE-A1- 10 063 288
- US-A1- 2008 261 927

## Description

### Field of invention

The present invention relates to the field of healthy edible products, especially to serum cholesterol lowering drinks.

### Background

Elevated serum total and/or LDL (low density lipoprotein) cholesterol levels are one of the major risk factors for cardiovascular disease. One of the first steps in improving the serum cholesterol profile is changes in life style including changes in diet and exercise. Food products enriched with components having cholesterol lowering effect beyond normal nutrition have been commercially available for some time. Representative examples are food products with added plant sterols and/or plant stanols. Food products that contain added plant sterols and/or plant stanols are widely available and include for example margarines, spreads, dairy products such as yoghurts, and bread. Also drinks containing plant sterols and/or plant stanols are available.

During recent years single-dose shot drinks containing plant sterols and/or plant stanols have become popular. In these drinks the daily dose of plant sterols and/or plant stanols is incorporated in a small volume drink, usually about 100 ml or even less. These drinks are typically based on dairy milk, drinkable yoghurt or soy milk with added flavouring agents (such as fruits, berries), sweetening agents, and plant sterols and/or plant stanols. These drinks contain plant sterols and/or plant stanols either in esterified form, i.e. as fatty acid esters, or in free form.

WO2010/084240 discloses beverages containing plant stanol ester or plant sterol ester. Although this document mentions that the products can be used as in-between meal beverages it has now been shown that the cholesterol lowering effect under such conditions is not optimal.

A recent meta-analysis (Demonty et al., J. Nutr. 139 (2009) 271-284) pointed out that plant sterols and plant stanols had better cholesterol lowering efficacy when incorporated in solid foods, such as margarines or yoghurts, than in liquid foods, such as drinks. For liquid food formats, such as drinks, simultaneous ingestion of a solid meal is needed to ensure the optimal cholesterol lowering efficacy. Thus, the consumers are usually advised to use the products containing plant sterols and/or plant stanols together with a meal for best cholesterol lowering efficacy.

Doornbos et al. (Eur. J. Clin. Nutr. (2006) 60(3):325-33) studied the cholesterol lowering efficacy of single-dose plant sterol enriched yoghurt drinks at two different consumption conditions - when consumed with a meal (as a part of the lunch) or without a meal (before breakfast). The LDL cholesterol lowering efficacy of the drink was not optimal if consumed without a meal.

Currently consumers are advised to consume one bottle per day of the single-dose shot drinks containing plant sterols and/or plant stanols together with a meal to ensure the optimal cholesterol lowering efficacy. However, many consumers would prefer to consume their single-dose shot drinks as a snack, without a meal. With the currently known formulations, the cholesterol lowering efficacy is not optimal if taken without a meal. The present invention provides drinks containing plant sterols and/or plant stanols, wherein the drinks are suitable to be consumed as a snack and still have an ensured serum LDL cholesterol lowering efficacy.

### Summary of the invention

The invention relates to a drink portion with serum LDL cholesterol lowering effect. The drink portion comprises 0.8-15 g total plant sterol and plant stanol equivalents, of which equivalents 0.10-3.0 g are in free form, provided that 5.0-25 % of the equivalents are in free form, and the rest of the equivalents are in esterified form. The drink portion further comprises 2.5-15 g triglyceride fat, at least one additional edible ingredient, and water.

The invention also relates to a drink with serum LDL cholesterol lowering effect. The drink comprises 0.50-10 w/v-% (% by weight per volume) total plant sterol and plant stanol equivalents, of which equivalents 5.0-25 % are in free form, and the rest of the equivalents are in esterified form. The drink further comprises 2.0-12 w/v-% triglyceride fat, at least one additional edible ingredient, and 60-97 w/v-% water.

The invention further relates to a pack containing at least one drink portion or the drink disclosed above.

The invention still further relates to a powder suitable for making a drink. The powder contains the dry edible ingredients of the drink portion according to the invention per portion of powder, or contains the dry edible ingredients at the same ratios as in the drink according to the invention.

The invention still further relates to a drink, drink portion or a powder of the invention for use as a medicament for lowering serum LDL cholesterol.

### Detailed description of the invention

The present invention discloses improved drinks with an ensured serum LDL cholesterol lowering effect. The "ensured" serum LDL cholesterol lowering effect means that the serum LDL cholesterol lowering effect that is obtained when the drink portion or drink according to the invention is consumed without a meal, is at least 70 % of the serum LDL cholesterol lowering effect of the same amount of total plant sterol and plant stanol equivalents consumed together with a meal. Preferably the serum LDL cholesterol lowering effect of the drink portion or drink ingested without a meal is at least 75 %, more preferably at least 80 %, still more preferably at least 85 %, further more preferably at least 90 %, even further more preferably at least 95% and most preferably at least 100 % of the effect of the same amount of total plant sterol and plant stanol equivalents taken together with a meal. The serum LDL cholesterol lowering efficacy with any daily intake of total plant sterol and/or plant stanol equivalents taken together with a meal can be calculated based on dose-response equations published by Demonty et al. (J. Nutr. 139 (2009) 271-284). Preferably the ensured serum LDL cholesterol lowering effect means that the serum LDL cholesterol lowering effect is maintained regardless of the consumption time of the drink portion or drink in relation to ingesting a meal.

By "snack" is in this specification meant an edible product to be ingested between meals (breakfast, lunch or dinner).

The invention relates to a drink portion with serum LDL cholesterol lowering effect, wherein the drink portion comprises
- 0.8-15 g, preferably 1.0-15 g, more preferably 1.5-12 g, still more preferably 1.8-10 g, and most preferably 2.0-8.0 g total plant sterol and plant stanol equivalents, of which equivalents
a) 0.10-3.0 g, preferably 0.15-3.0 g, more preferably 0.18-2.5 g, still more preferably 0.20-2.0 g, and most preferably 0.20-1.5 g are in free form, provided
   that 5.0-25 %, preferably 6.0-23 %, more preferably 7.0-20 %, and still more preferably 8.0-18 %, and most preferably 10-15 % by weight are in free form, and
b) the rest of the equivalents are in esterified form, - 2.5-15 g, preferably 3.0-12 g, more preferably 4.0-12 g, still more preferably 4.5-12 g, even more preferably 5.0-12 g, and most preferably 6.0-12 g triglyceride fat, - at least one additional edible ingredient, and - water.

The invention also relates to a drink with serum LDL cholesterol lowering effect, wherein the drink comprises
- 0.50-10 w/v-%, preferably 0.80-10 w/v-%, more preferably 1.0-10 w/v-%, still more preferably 1.2-10 w/v-%, even more preferably 1.5-10 w/v-% and most preferably 2.0-10 w/v-% total plant sterol and plant stanol equivalents, of which equivalents by weight
   a) 5.0-25 %, preferably 6.0-23 %, more preferably 7.0-20 %, and still more preferably 8.0-18 %, and most preferably 10-15 % are in free form, and
   b) the rest of the equivalents are in esterified form,
- 2.0-12 w/v-%, preferably 3.0-12 w/v-%, more preferably 4.0-12 w/v-%, still more preferably 4.5-12 w/v-%, even more preferably 5.0-12 w/v-%, and most preferably 6.0-12 w/v-% triglyceride fat,
- 0.5-37.5 w/v-% at least one additional edible ingredient, and
- 60-97 w/v-% water.

By "w/v-%" is here meant percent by weight per volume (1 w/v-% = 1 g/100 ml).

By "drink" is in this specification meant a food product meant to be consumed by drinking, i.e. the producer has designed the product for drinking. Examples of such products are
- dairy drinks such as milk, various milk drinks (such as smoothies, milkshakes, soured milk drinks and drinkable yoghurts (i.e. fermented milk drinks)),
- non-dairy drinks (meaning milk alternative drinks made from e.g. soy, rice, oat, almond and/or wheat instead of dairy milk) such as non-dairy milk, non-dairy smoothies and drinkable non-dairy yoghurts,
- fruit, berry and/or vegetable drinks,
- coffee, cocoa and/or tea drinks,
- sport drinks,
- cloudy soft drinks, and
- healthy drinks (can be any of the previous ones listed) containing e.g. vitamins, fibres, minerals, probiotics and/or antioxidants.

The drink according to the invention is aimed to have an ensured serum LDL cholesterol lowering effect also when used as a snack i.e. without ingesting a meal. This means that e.g. one portion can be consumed as a snack drink i.e. not accompanied by a meal at the same time. A portion can therefore suitably be consumed at a consumption occasion as defined herein and still give an ensured LDL cholesterol lowering effect.

By "consumption occasion" is in this specification meant the time used for ingesting the drink or drink portion according to the invention (e.g. from 5 seconds to 30 minutes), as well as the time during which no meal is ingested before starting the ingestion of the drink or drink portion (e.g. at least 30 minutes) and the time during which no meal is ingested after ending the ingestion of the drink or drink portion (e.g. at least 30 minutes). The duration of the consumption occasion is therefore practically e.g. from 1 hour to 1.5 hours. The benefit obtained from the present invention is that the drink portion or drink can be ingested at such a consumption occasion and still give an ensured LDL cholesterol lowering effect.

The portion size of the drink is suitable to be consumed within 30 minutes at most. The portion size of the drink is preferably 50-500 ml, more preferably 50-400 ml, still more preferably 65-300 ml, even more preferably 100-300 ml, and most preferably 125-300 ml.

The drink may of course also be consumed together with (i.e. including also immediately before or after) a meal or together with other snack products, but the aim of this invention is that the consumer obtains the ensured serum LDL cholesterol lowering effect even when consuming the drink according to the present invention without a meal. The trader of the drink can therefore advise the consumer that the drink can be used as a snack. The drink according to the invention could therefore preferably be named a snack drink.

By "drink portion" is meant the amount of drink meant to be consumed at one consumption occasion, i.e. the drink portion corresponds to a drink serving. Preferably the size of the drink portion according to the invention is 50-500 ml, more preferably 50-400 ml, still more preferably 65-300 ml, even more preferably 100-300 ml, and most preferably 125-300 ml.

The drink portion and the drink according to the invention have an ensured serum LDL cholesterol lowering effect also when used as snacks. The consumer can therefore be advised that the drink portion and the drink can be used as snacks. The drink portion and the drink according to the invention have at least 70 % of the serum LDL cholesterol lowering effect when consumed without a meal compared to when the same amount of total plant sterol and plant stanol equivalents are consumed with a meal. Preferably, the drink portion as well as the drink maintain the LDL cholesterol lowering efficacy regardless of the consumption time of the drink or drink portion in relation to ingesting a meal. Therefore, the drink portion or drink according to the invention is suitable to be ingested at a consumption occasion as defined herein. Most preferably the drink is a snack and the drink portion is a snack.

By the term "edible ingredient" is here meant all components of the drink or drink portion e.g. plant sterols and/or plant stanols, triglyceride fat, water and additional edible ingredients.

In this specification plant sterols include and preferably are 4-desmethyl sterols and 4-monomethyl sterols, and plant stanols include and preferably are 4-desmethyl stanols and 4-monomethyl stanols. Typical 4-desmethyl sterols are sitosterol, campesterol, stigmasterol, brassicasterol, 22-dehydro-brassicasterol and δ5-avenasterol. Typical stanols are sitostanol, campestanol and their 24-epimers. The term "plant sterols and/or plant stanols" includes all possible mixtures of sterols and/or stanols as well as any individual plant sterol and/or plant stanol.

The plant sterols and/or plant stanols used in this invention contain plant sterols and/or plant stanols in both free and esterified form. The plant sterols and/or plant stanols that are in esterified form, are esterified with a carboxylic acid or with a mixture of carboxylic acids. Examples of suitable carboxylic acids are fatty acids (having 2-24 carbon atoms, being saturated, monounsaturated or polyunsaturated, including also special fatty acids, such as EPA and DHA, and conjugated fatty acids, e.g. CLA). Preferably the plant sterols and/or plant stanols are esterified with fatty acids, most preferably with vegetable oil based fatty acids.

Plant stanol fatty acid ester and the effects thereof, as well as a suitable method for its preparation, are disclosed in US 6 174 560. Obviously also plant sterol esters can efficiently be produced by the production method disclosed in US 6 174 560. Alternatively fatty acid esters of plant sterols and/or plant stanols can be produced by any method disclosed in the art.

By "plant sterol equivalents" is meant the amount of plant sterols calculated as the amount of free plant sterols. This means that in case of plant sterol ester, only the plant sterol part of the molecule is calculated as a plant sterol equivalent and the acid part is ignored.

Correspondingly, by "plant stanol equivalents" is meant the amount of plant stanols calculated as the amount of free plant stanols. This means that in case of plant stanol ester, only the plant stanol part of the molecule is calculated as a plant stanol equivalent and the acid part is ignored.

By "total plant sterol and plant stanol equivalents" is thus meant the total amount of plant sterol equivalents and plant stanol equivalents. The "total plant sterol and plant stanol equivalents" therefore include plant sterols and plant stanols in both free and esterified form, however, expressed as the total amount of free plant sterols and free plant stanols. Thus, the equivalents may contain only plant sterols or only plant stanols or their mixtures.

Of the total plant sterol and plant stanol equivalents 5.0-25 % by weight, preferably 6.0-23 % by weight, more preferably 7.0-20 % by weight, still more preferably 8.0-18 % by weight, and most preferably 10-15 % by weight are in free form, and the rest of the equivalents are in esterified form.

The plant sterols and/or plant stanol composition used in the present invention may be obtained by stopping the esterification reaction when a desired degree of esterification has been achieved. Alternatively, in order to prepare the plant sterols and/or plant stanols, a suitable amount of free plant sterols and/or free plant stanols can be added to plant sterol ester and/or plant stanol ester which has been made e.g. utilising any conventional esterification method in which a high degree of esterification (typically at least 97 % by weight) can be obtained.

The plant sterols and/or plant stanols can be incorporated into the drink or drink portion at any suitable production step in the preparation of the drink or drink portion. The free plant sterols and/or plant stanols and the esterified plant sterols and/or plant stanols can also be added separately at any suitable production step. In that case the composition of plant sterols and/or plant stanols is at the latest formed within the final drink or drink portion.

Most preferred in drinks or drink portions according to the present invention is a composition of plant sterols and/or plant stanols in which 50-100 % (by weight) of the total plant sterol and plant stanol equivalents are plant stanol equivalents. Preferably there is at least 70 %, more preferably at least 80 %, still more preferably at least 90 %, and most preferably at least 95 % by weight plant stanol equivalents, of the total plant sterol and plant stanol equivalents.

Still preferred in drinks or drink portions according to the present invention is a composition of plant sterols and/or plant stanols in which 50-100 % (by weight) of the plant sterols and/or plant stanols in free form is plant sterol. Preferably, of the total amount of free plant sterols and free plant stanols, at least 70 %, more preferably at least 80 %, still more preferably at least 90 %, and most preferably at least 95 % by weight are free plant sterols. It is, however, also possible to have a composition in which 50-100 % by weight of the free plant sterols and/or plant stanols is free plant stanol.

The drink portion according to the invention contains plant sterols and/or plant stanols in an amount of
- 0.8-15 g, preferably 1.0-15 g, more preferably 1.5-12 g, still more preferably 1.8-10 g, and most preferably 2.0-8.0 g total plant sterol and plant stanol equivalents, of which equivalents
   a) 0.10-3.0 g, preferably 0.15-3.0 g, more preferably 0.18-2.5 g, still more preferably 0.20-2.0 g, and most preferably 0.20-1.5 g are in free form, provided that 5.0-25 %, preferably 6.0-23 %, more preferably 7.0-20 %, and still more preferably 8.0-18 %, and most preferably 10-15 % by weight are in free form, and
   b) the rest of the equivalents are in esterified form.

The drink according to the invention contains plant sterols and/or plant stanols in an amount of
- 0.50-10 w/v-%, preferably 0.80-10 w/v-%, more preferably 1.0-10 w/v-%, still more preferably 1.2-10 w/v-%, even more preferably 1.5-10 w/v-% and most preferably 2.0-10 w/v-% total plant sterol and plant stanol equivalents, of which equivalents
   a) 5.0-25 %, preferably 6.0-23 %, more preferably 7.0-20 %, and still more preferably 8.0-18 %, and most preferably 10-15 % are in free form, and
   b) the rest of the equivalents are in esterified form.

As used here, by "triglyceride fat" is meant edible fats and oils that consist mainly (at least 90, preferably at least 95 and most preferably at least 98 % by weight) of triacylglycerols. Triglyceride fat can be an intrinsic part of the drink portion and drink (i.e. triglyceride fat of soybean oil in soy based drinks or dairy triglyceride fat in milk based drinks) or it can be separately added to the drink portion or drink. Triglyceride fat can also be a mixture of different edible fats and/or oils. Typical examples of triglyceride fats are vegetable oils such as canola/rapeseed oil, soybean oil, sunflower oil, olive oil, corn oil, sesame seed oil, cottonseed oil, wheat germ oil, oat oil, linseed oil, peanut oil, camelina oil as well as triglyceride fat of dairy and fish origin. The triglyceride fats can be naturally occurring or modified, for example hydrogenated, transesterified or contain structured triacylglycerols. Preferably the triglyceride fats are vegetable oils, most preferably vegetable oils containing omega-3 fatty acids. Preferably the amount of saturated fatty acids in the triglyceride fat is less than 35 % of the total fatty acids, more preferably less than 30 %, still more preferably less than 25 %, and most preferably less than 20 % of the total fatty acids. Preferably, at least part of the triglyceride fat is an intrinsic part of the drink portion and drink.

The drink according to the present invention contains 2.0-12 %, preferably 3.0-12 %, more preferably 4.0-12 %, still more preferably 4.5-12 %, even more preferably 5.0-12 %, and most preferably 6.0-12 % by weight per volume triglyceride fat.

The drink portion according to the invention contains 2.5-15 g, preferably 3.0-12 g, more preferably 4.0-12 g, still more preferably 4.5-12 g, even more preferably 5.0-12 g, and most preferably 6.0-12 g triglyceride fat.

The drink portion and drink according to the present invention also contain at least one additional (i.e. further) edible ingredient, such as an emulsifier, a stabiliser, a protein, a sweetening agent, a flavouring agent, a colouring agent, a preservative, and an ingredient with beneficial health effect, or mixtures of additional edible ingredients. The amount of the additional edible ingredient(s) in the drink according to the invention is preferably from 0.5 to 37.5 w/v-%. The additional edible ingredient can be an intrinsic part of the drink portion or drink (e.g. protein in soy or dairy based drinks) and/or it can be separately added to the drink portion or drink.

With the term "emulsifier" is here meant a substance promoting the formation of the emulsion. Non-limiting examples of suitable emulsifiers that can be used in this invention are mono- and diglyceride derivatives, such as acetic, lactic, succinic or citric acid esters; sorbitan esters; polysorbates; stearoyl lactylates, such as sodium stearoyl lactylate and calcium stearoyl lactylate; diacetyl tartaric acid esters; diacetyl lactic acid esters; sugar esters; lecithin and its derivatives, and mixtures of any of these. Preferred emulsifiers are citric acid esters of mono- and diglycerides, lactic acid esters of mono- and diglycerides, diacetyl-tartaric acid esters of mono- and diglycerides, polysorbates, sodium stearoyl lactylate, calcium stearoyl lactylate, sucrose fatty acid esters and mixtures of any of these. Most preferable emulsifiers used in the present invention are citric acid ester, lactic acid ester and diacetyl tartaric acid ester of mono- and diglycerides, polysorbates and mixtures of any of these. The amount of emulsifier used in the drink portion and drink according to the present invention, if any, is typically from 0.05 to 0.50 % by weight per volume, and more typically from 0.10 to 0.40 % by weight per volume of the drink portion and drink.

With the term "stabiliser" is here meant a substance promoting the stability of the drink portion and drink. Suitable examples include hydrocolloids such as pectin, carrageenan, starches and modified starches, maltodextrins, alginates, various gums such as xanthan, guar, locust bean, tragacanth, arabic and gellan gums, and mixtures thereof. A skilled person is well aware of suitable amounts of different stabilisers to be used in order to obtain a commercially acceptable drink portion and drink.

The drink portion and drink of the present invention may contain protein. Examples of suitable proteins include dairy proteins, such as casein and whey protein; soy protein, various cereal proteins, such as wheat or rice proteins, pea protein, lupin protein, canola protein and mixtures of any of these. The protein can be an intrinsic part of the drink portion and drink (i.e. soybean proteins in a soy based drink or dairy proteins in a milk based drink) or can be separately added to them e.g. as flour containing protein, protein concentrate or protein isolate. Preferably the drink portion and drink contain non-dairy protein.

The protein content of the drink is preferably from 1.0 to 12 % by weight per volume of the drink, more preferably 3.0-11 %, still more preferably 4.0-11 %, even more preferably 5.0-11 %, further more preferably 6.0-11 %, and most preferably 6.0-9.0 % by weight per volume of the drink. This protein content is suitable for any portion size (50-500 ml) of the drink according to the invention.

The drink portion according to the invention may contain 1.0-35 g, preferably 2.0-33 g, more preferably 3.0-30 g, still more preferably 4.0-28 g protein. Typically there is at least 6.0 g, more typically at least 8.0 g, still more typically at least 10 g, even more typically at least 12 g, and most typically at least 14 g protein per drink portion.

Preferably, the drink portion and drink according to the invention are lactose-free or have only a low level of lactose. By low level of lactose is here meant drinks having at most 1 g lactose per 100 ml drink. Non-dairy drinks, such as soy, oat and rice based drinks are naturally lactose-free and thus are very suitable for the present invention. Suitable are also dairy milk based drinks having low levels of lactose or ones that are lactose-free. However, dairy drinks containing a natural or increased level of lactose are also suitable alternatives.

The drink portion and drink according to the present invention may contain various sweetening agents and flavouring agents. As used here the term "sweetening agent" includes compounds used to increase the sweetness of the product. Non-limiting examples of these are e.g. sucrose and other sugars, sugar syrups, honey, sugar alcohols such as xylitol, maltitol, lactitol, sorbitol and erythritrol, non-carbohydrate sweeteners, such as aspartame, acesulfame-K, saccharin, cyclamates, sucralose and stevia. As used here the term "flavouring agent" means ingredients that bring flavour to the drink portion and drink. Examples of these include various plant extracts; aromas such as vanilla; cocoa powder; coffee; tea; and preparations from berries, fruits and/or vegetables such as jam, jelly, juice, puree, juice concentrate, as well as berries and/or fruits as such (e.g. fresh, deep-frozen or dried).

Non-limiting examples of ingredients with beneficial health effects are vitamins (e.g. C, D, E and K vitamins), minerals, fibres, antioxidants, polyphenols and probiotics.

The drink portion and drink according to the present invention also contain water. The term "water" includes also water being naturally present in any edible ingredient of the drink portion or drink, such as the water content of milk or of a fruit preparation. The water content of the drink portion and drink according to the invention is typically 60-97 %, preferably at least 65 %, more preferably at least 70 %, and most preferably at least 75 % by weight per volume. This water content is suitable for any portion size (50-500 ml) of the drink according to the invention. The drink portion and drink according to the present invention are an oil in water (O/W) emulsion. By "oil in water (O/W) emulsion" is in this specification meant emulsions in which oil is present as the dispersed phase and water as the continuous phase. The drink portion and drink of the present invention may be an acid stable O/W emulsion. By acid stable emulsion is here meant O/W emulsions that are stable in acidic conditions, i.e. oil/fat and water phases do not separate despite low pH. Especially, this means emulsions that retain their structure at pH values of typically 5.0, 4.5, 4.0, 3.5, 3.0, or even 2.5. The structure can be measured by assessing the droplet (or particle) size distribution of the emulsion by known techniques, e.g. by light scattering techniques. Droplet size distribution of the emulsion remains unchanged or there are only minor changes of the droplet size distribution when the emulsion is exposed to acidic conditions compared to the original droplet size distribution of the emulsion.

The structural properties of drink emulsions change when they are ingested into the mouth and gastrointestinal tract. The emulsions are subjected to very low pH, mechanical agitation and digestive juices in the stomach. The pH-value is typically 1-3 in human stomach. By acid stable emulsions is preferably meant emulsions that retain their structure in the typical pH values of human stomach.

The carbohydrate content of the drink portion and drink is 0-25 w/v-% (g/100 ml), and preferably there is at least 1.0 w/v-%, more preferably at least 2.0 w/v-%, still more preferably at least 4.0 w/v-%, even more preferably at least 6.0 w/v-%, and most preferably at least 8.0 w/v-%. This carbohydrate content is suitable for any portion size (50-500 ml) of the drink according to the invention.

The energy content of the drink portion and drink according to the invention is preferably at least 250 kJ/100 ml, more preferably at least 300 kJ/100 ml, still more preferably at least 350 kJ/100 ml, even more preferably at least 400 kJ/100 ml, further more preferably at least 450 kJ/100 ml, still further more at least 500 kJ/100 ml and most preferably at least 550 kJ/100 ml. The energy content of the drink portion and drink is preferably at most 1300 kJ/100 ml, more preferably at most 1000 kJ/100 ml, still more preferably at most 800 kJ/100 ml, and most preferably at most 700 kJ/100 ml. These energy contents are suitable for any portion size (50-500 ml) of the drink according to the invention.

The invention further relates to a pack containing the drink or at least one drink portion with serum LDL cholesterol lowering effect as disclosed above.

By "pack" is here meant the physical protection within which the drink or at least one drink portion is enclosed. The pack can be e.g. a plastic bottle or any container suitable for liquid food products. The pack may contain one or more drink portions, but most preferably it contains one drink portion.

Preferably the size of the pack is from 50 ml to 25 l, more preferably from 50 ml to 1.5 l, still more preferably from 50 ml to 1 l, even more preferably from 50 to 500 ml, still even more preferably from 50 to 400 ml, further more preferably from 65 to 300 ml, still further preferably from 100 to 300 ml, and most preferably from 125 to 300 ml.

Preferably the size of the drink portion in the pack according to the invention is 50-500 ml, more preferably 50-400 ml, further more preferably 65-300 ml, still more preferably 100-300 ml, and most preferably 125-300 ml.

According to the invention the pack preferably contains one drink portion.

The pack may further deliver the advice to the consumer that the drink portion has an ensured serum LDL cholesterol lowering effect also when used as a snack. This means that the drink portion has at least 70 % of the serum LDL cholesterol lowering effect when consumed without a meal compared to when the same amount of total plant sterol and plant stanol equivalents are consumed with a meal. The drink portion preferably maintains the LDL cholesterol lowering efficacy regardless of the consumption time thereof in relation to ingesting a meal. Most preferably the pack according to the invention can deliver the advice to the consumer that the drink portion can be used as a snack.

According to one preferred embodiment of the invention the drink has a portion size from 200 ml to 300 ml and the drink comprises - 0.50-7.5 w/v-%, preferably 0.80-6.0 w/v-%, more preferably 1.0-5.0 w/v-%, and most preferably 1.2-4.0 w/v-% total plant sterol and plant stanol equivalents, of which equivalents
a) 5.0-25 %, preferably 6.0-23 %, more preferably 7.0-20 %, and still more preferably 8.0-18 %, and most preferably 10-15 % are in free form, and
b) the rest of the equivalents are in esterified form,
   - 2.0-7.5 w/v-%, preferably 2.5-7.0 w/v-%, more preferably 3.0-6.5 w/v-%, and most preferably 4.0-6.0 w/v-% triglyceride fat,
   - 0.5-37.5 w/v-% at least one additional edible ingredient, and
   - 60-97 w/v-% water.

According to a second preferred embodiment of the invention the drink has a portion size from more than 125 ml to less than 200 ml (such as from 126 ml to 199 ml) and the drink comprises
- 0.50-10 w/v-%, preferably 1.0-10 w/v-%, more preferably 1.2-8.0 w/v-%, most preferably 1.5-6.0 w/v-% total plant sterol and plant stanol equivalents, of which equivalents
   a) 5.0-25 %, preferably 6.0-23 %, more preferably 7.0-20 %, and still more preferably 8.0-18 %, and most preferably 10-15 % are in free form, and
   b) the rest of the equivalents are in esterified form,
- 2.0-12 w/v-%, preferably 2.5-10 w/v-%, more preferably 3.0-8.0 w/v-%, and most preferably 4.0-6.0 w/v-% triglyceride fat,
- 0.5-37.5 w/v-% at least one additional edible ingredient, and
- 60-97 w/v-% water.

According to a third preferred embodiment of the invention the drink has a portion size from 65 ml to 125 ml and the drink comprises
- 0.80-10 w/v-%, preferably 1.0-10 w/v-%, more preferably 1.5-10 w/v-% and most preferably 2.0-8.0 w/v-% total plant sterol and plant stanol equivalents, of which equivalents
   a) 5.0-25 %, preferably 6.0-23 %, more preferably 7.0-20 %, and still more preferably 8.0-18 %, and most preferably 10-15 % are in free form, and
   b) the rest of the equivalents are in esterified form,
- 2.0-12 w/v-%, preferably 3.0-12 w/v-%, more preferably 4.0-10 w/v-%, most preferably 5.0-8.0 w/v-% triglyceride fat,
- 0.5-37.2 w/v-% at least one additional edible ingredient, and
- 60-97 w/v-% water.

For these three embodiments the additional edible ingredient(s) of the drink is (are) as disclosed herein before. A pack suitable for these three preferred embodiments is as disclosed herein before and furthermore the size of the pack is still more preferably from 200 ml to 300 ml, from more than 125 ml to less than 200 ml, and from 65 ml to125 ml, respectively.

The invention still further relates to a powder suitable for making a drink. The powder contains, preferably consists of, the dry edible ingredients of the drink portion according to the invention per portion of powder, or the dry edible ingredients at the same ratios as in the drink according to the invention (i.e. dry edible ingredients in the same amount in relation to each other as in the drink according to the invention).

Such a drink powder contains the dry ingredients of the drink portion or the drink. The drink powder may be prepared by drying the drink portion or the drink by any suitable drying technique, e.g. by spray drying. In the case of spray drying, it is favourable to have protein and/or maltodextrin as one of the additional edible ingredient(s) in the drink portion or the drink. To reconstruct the drink portion or the drink, the drink powder is mixed with liquid, such as e.g. water. It is also possible to reconstruct the drink portion or the drink by mixing the drink powder with some other liquid than that which was originally removed by drying. For example, a dairy drink portion or drink can be dried to form a drink powder, which can be added e.g. into coffee to reconstruct the composition of the drink portion or the drink.

A method for lowering serum LDL cholesterol in a subject in need thereof is also disclosed, wherein the subject ingests a drink or drink portion according to the invention, without ingesting a meal. This means that the method of lowering LDL cholesterol is improved in that way that the drink or drink portion can be consumed without ingesting a meal at the same time. This means that the drink portion or drink can be ingested as a snack drink, i.e. not accompanied by a meal at the same time of consumption. The method therefore enables an ensured lowering of serum LDL cholesterol without ingesting a meal at the same consumption occasion, e.g. not ingesting a meal within 30 minutes before starting and 30 minutes after ending the consumption of the drink or drink portion. The aim of the method is that the subject obtains the ensured serum LDL cholesterol lowering effect even when using the drink or drink portion according to the present invention without a meal.

### Example 1 (comparative example)

Two drinks were prepared. The recipes of the drinks are presented in Table 1. Both drinks contained 2.0 g plant stanol equivalents / 100 ml drink. In Drink 1, 10 % (0.2 g) of the plant stanol equivalents were free plant stanols and 90 % (1.8 g) were plant stanols esterified to fatty acids. The reference drink (C700) contained commercial plant stanol ester wherein plant stanols were esterified with rapeseed oil fatty acids. (Classic C700, Raisio Nutrition Ltd.).

**Table 1.**

| Ingredient g/100 ml | Drink 1 | Drink C700 |
|---|---|---|
| Soy milk (2.7 % fat) | 56.0 | 56.0 |
| Plant stanol composition | 3.2 | - |
| Plant stanol ester Classic C700 | - | 3.3 |
| Sugar | 2.0 | 2.0 |
| Carrageenan | 0.03 | 0.03 |
| Aspartame | 0.02 | 0.02 |
| Emulsifiers | 0.42 | 0.42 |
| Sorbate | 0.12 | 0.12 |
| Color | 0.001 | 0.001 |
| Aromas (apple, strawberry) | 0.4 | 0.4 |
| Water to bring the volume to | 100 ml | 100 ml |

The dry ingredients and water were mixed into soy milk and warmed up to 60 °C. After that the plant stanol composition or the Plant stanol ester Classic C700, and emulsifiers were mixed together and then added to the warmed mixture. The obtained mixture was UHT treated (145 °C, 3-5 s), homogenised (200 bar, 60 °C) and packaged.

The content of triglyceride fat, total plant sterol and plant stanol equivalents, and free plant sterols and plant stanols are presented in Table 2. The content of total plant sterol and plant stanol equivalents was analysed by saponifying the sample with alcoholic potassium hydroxide, extracting the unsaponifiable matter with hexane, and analysing as silyl ether derivatives by a gas chromatographic method. Free plant sterols and plant stanols were analysed by using solid phase extraction and gas chromatographic analysis. The amount of plant sterol and plant stanol equivalents in esterified form was calculated by subtracting the amount of free plant sterols and plant stanols from the amount of total plant sterol and plant stanol equivalents. The amount of plant sterol ester and plant stanol ester was calculated by multiplying the amount of plant sterol and plant stanol equivalents in esterified form by 1.67. The crude fat content was analysed by acid hydrolysis and Büchi extraction. From the crude fat content the amount of free plant sterols and plant stanols and the amount of plant sterol ester and plant stanol ester were subtracted to obtain the content of triglyceride fat.

**Table 2.**

| The content of triglyceride fat, total plant sterol and plant stanol equivalents, and free plant sterols and plant stanols (g/100 ml) | Drink 1 | Drink C700 |
|---|---|---|
| Triglyceride fat | 1.5 | 1.5 |
| Plant stanol equivalents* | 2.0 | 2.0 |
| Free plant stanols | 0.2 | 0.0 |
| *including both esterified and free plant stanols | | |

The cholesterol lowering efficacy of the drinks was studied in mildly hypercholesterolemic subjects (n=35/group). The subjects consumed either drink 1 (100 ml) or drink C700 (100 ml) separately from a meal, in the morning, minimum 30 min before breakfast meal, for 3 weeks. No other beverages like coffee or tea were allowed within 30 minutes from taking the drinks. The study subjects were requested to keep the time of intake of the study drink constant during the whole intervention. Otherwise, the subjects followed their habitual diet. Blood samples were taken after 10-12 h overnight fast, both before starting the consumption of the drink and after consuming the drink for 3 weeks. Serum total cholesterol, HDL cholesterol and triglycerides were analysed by enzymatic methods and LDL cholesterol (the primary outcome of the study) was calculated with Friedewald's formula.

The results of the clinical study are presented in Table 3. The LDL cholesterol lowering efficacy of the drink 1 was compared to the efficacy obtained with Drink C700 and also to the prior art study by Doornbos et al. (2005). The study design in the Doornbos study was comparable, i.e. small volume (100 ml) yoghurt drinks containing plant sterol ester were taken 1/2 hour before breakfast on an empty stomach. The triglyceride fat (1.5 g dairy fat) content of Doornbos drink B was comparable to that of drink 1, but the plant sterol content (2.8 g total plant sterol and plant stanol equivalents) was higher than the total plant sterol and plant stanol equivalents in the drink 1. As there was no placebo drink taken without a meal in the Doornbos study, the LDL cholesterol lowering from the baseline level was used for the comparative purposes.

**Table 3.**

| | LDL cholesterol mmol/l Baseline | LDL cholesterol mmol/l End of intervention | Change mmol/l from baseline | Change % from baseline |
|---|---|---|---|---|
| Drink 1 | 4.01 | 3.90 | -0.11 | -2.7 |
| Drink C700 | 4.11 | 3.98 | -0.13 | -3.2 |
| Doornbos | 4.06 | 3.91 | -0.15 | -3.7 |
| study, drink B | | | | |

The LDL cholesterol was lowered only by 2.7 % from the baseline level in the subjects consuming the drink 1 without a meal, 1/2 hour before breakfast, on an empty stomach. The LDL cholesterol lowering efficacy was comparable to the efficacy obtained with the drink C700 and the drink B in the study of Doornbos et al. (2005). Increasing the amount of free plant stanols did not thus improve the LDL cholesterol lowering efficacy.

### Example 2

Two drinks (drink 2 and drink 3) were prepared. The recipes of the drinks are presented in Table 4. Both drinks contained 2.0 g plant stanol equivalents /100 ml drink and an increased level of triglyceride fat (6.0 g triglyceride fat, from soy milk and rapeseed oil) as compared to the drinks of Example 1. Drink 2 contained commercial plant stanol ester Classic C700. Drink 3 contained 2.0 g plant stanol equivalents, of which 10 % (0.2 g) were free plant stanols and 90 % (1.8 g) were plant stanols esterified to fatty acids.

**Table 4.**

| Ingredient g/100 ml | Drink 2 | Drink 3 |
|---|---|---|
| Soy milk (2.7 % fat) | 87.3 | 87.3 |
| Plant stanol composition | | 3.2 |
| Plant stanol ester Classic C700 | 3.3 | |
| Rapeseed oil | 3.6 | 3.6 |
| Sugar | 2.0 | 2.0 |
| Carrageenan | 0.03 | 0.03 |
| Aspartame | 0.02 | 0.02 |
| Emulsifiers | 0.42 | 0.42 |
| Sorbate | 0.12 | 0.12 |
| Color | 0.001 | 0.001 |
| Aromas (apple, strawberry) | 0.4 | 0.4 |
| Water to bring the volume to | 100 ml | 100 ml |

Drink 2 and Drink 3 were prepared and analysed in a similar way as the drinks in Example 1. The content of triglyceride fat, total plant sterol and plant stanol equivalents, and free plant sterols and plant stanols of Drink 2 and Drink 3 are presented in Table 5.

**Table 5.**

| The content of triglyceride fat, total plant sterol and plant stanol equivalents, and free plant sterols and stanols (g/100 ml) | Drink 2 | Drink 3 |
|---|---|---|
| Triglyceride fat | 6.0 | 6.0 |
| Plant stanol equivalents* | 2.0 | 2.0 |
| Free plant stanols | 0.0 | 0.2 |
| *including both esterified and free plant stanols | | |

The cholesterol lowering efficacy of the drinks 2 and 3 was studied in a same way as the efficacy of the drinks in Example 1. The results are presented in Table 6.

**Table 6.**

| | LDL cholesterol mmol/l Baseline | LDL cholesterol mmol/l End of intervention | Change mmol/l from baseline | Change % from baseline |
|---|---|---|---|---|
| Drink 2 | 3.55 | 3.43 | -0.12 | -3.4 |
| Drink 3 | 3.49 | 3.24 | -0.25 | -7.2 |

Increasing the amount of triglyceride fat (Drink 2) did not improve the LDL cholesterol lowering efficacy compared to Drink C700 of Example 1. The LDL cholesterol was lowered only by 3.4 % from the baseline level in the subjects consuming Drink 2 without a meal, 1/2 hour before breakfast, on an empty stomach. However, it was surprisingly found that increasing both the triglyceride fat content and the content of free plant stanols (Drink 3), good LDL cholesterol lowering efficacy was achieved even when the drinks were consumed in fasted state. The LDL cholesterol was lowered by 7.2 % from the baseline level in the subjects consuming the drink 3 without a meal, 1/2 hour before breakfast, on an empty stomach. The LDL cholesterol lowering efficacy was thus of the same magnitude that can be achieved when drinks containing about 2 g plant sterols and/or plant stanols are consumed together with a meal.

### Example 3

A drinkable yoghurt type drink was prepared of the following ingredients.

| Ingredient g/100 ml | |
|---|---|
| Milk (3.5 % fat) | 89.8 |
| Plant sterol and stanol composition* | 3.1 |
| Sugar | 2.0 |
| Pectin | 0.1 |
| Aromas | 0.02 |
| Yoghurt cultures | |
| Strawberry juice to bring the volume to 100 ml | |
| * Of the total plant sterol and plant stanol equivalents 85 % were esterified plant sterols and plant stanols, and 15 % were free plant sterols and plant stanols. Of the total plant sterol and plant stanol equivalents 53 % were plant stanols and 47 % plant sterols. Of the free plant sterols and plant stanols, 53 % were plant stanols and 47 % plant sterols. | |

The portion size of this drink was 150 ml. One portion contained 4.5 g triglyceride fat (dairy fat) and 3 g total plant sterol and plant stanol equivalents, of which 0.45 g was in free form.

### Example 4

A drink containing soy protein isolate was prepared of the following ingredients.

| Ingredient g/100 ml | |
|---|---|
| Soy protein isolate | 6.0 |
| Plant stanol composition* | 4.8 |
| Rapeseed oil | 6.3 |
| Fructose | 1.5 |
| Carrageenan | 0.1 |
| Emulsifier | 0.4 |
| Aromas (raspberry) | 0.1 |
| Water to bring the volume to 100 ml | |
| * Of the plant stanol equivalents 90 % were esterified and 10 % were free plant stanols. | |

The drink contained 6 g triglyceride fat (rapeseed oil) and 3 g plant stanol equivalents, of which 0.3 g were in free form, per 100 ml portion size. The protein content of the drink was 6 g/100 ml.

### Example 5

An oat based drink was prepared of the following ingredients.

| Ingredient g/100 ml | |
|---|---|
| Oat milk (0.9 % fat, 0.7 % protein) | 75.1 |
| Plant stanol composition* | 2.0 |
| Camelina oil | 1.8 |
| Sucrose | 4.0 |
| Pectin | 0.2 |
| Lingonberry juice | 7.0 |
| Soy protein isolate | 3.0 |
| Water to bring the volume to 100 ml | |
| * Of the plant stanol equivalents 90 % were esterified and 10 % were free plant stanols | |

The drink was packaged into 250 ml portion sizes. One portion (250 ml) contained 6.2 g triglyceride fat (camelina oil and the triglyceride fat from oat milk) and 3.1 g plant stanol equivalents, of which 0.3 g was in free form. The protein content was 1.6 g/portion.

### Example 6

A milk based drink was prepared of the following ingredients.

| Ingredient g/100 ml | |
|---|---|
| Plant stanol composition* | 3.8 |
| Sunflower oil | 5.8 |
| Cocoa powder | 1.0 |
| Sugar | 5.0 |
| Emulsifier | 0.3 |
| Carrageenan | 0.1 |
| Skim milk (0 % fat) to bring the volume to 100 ml | |
| * Of the plant stanol equivalents 82 % were esterified and 18 % were free plant stanols | |

The drink was packaged into 60 ml portion sizes. One portion (60 ml) contained 3.5 g triglyceride fat and 1.5 g plant stanol equivalents, of which 0.3 g was in free form.

### Example 7

A fruit juice based drink was prepared of the following ingredients.

| Ingredient g/100 ml | |
|---|---|
| Plant stanol composition* | 1.6 |
| Rapeseed oil | 2.5 |
| Sugar | 6.0 |
| Emulsifier | 0.1 |
| Pectin | 0.1 |
| Orange juice to bring the volume to 100 ml | |
| * Of the plant stanol equivalents 90 % were esterified and 10 % were free plant stanols | |

The drink was packaged into 500 ml bottles, containing two portions per bottle. One portion (250 ml) contained 6.25 g triglyceride fat and 2.5 g plant stanol equivalents, of which 0.2 g was in free form.

## Claims

1. A drink portion with serum LDL cholesterol lowering effect, wherein the drink portion comprises
- 0.8-15 g, preferably 1.0-15 g, more preferably 1.5-12 g, still more preferably 1.8-10 g, and most preferably 2.0-8.0 g total plant sterol and plant stanol equivalents, of which equivalents
a) 0.10-3.0 g, preferably 0.15-3.0 g, more preferably 0.18-2.5 g, still more preferably 0.20-2.0 g, and most preferably 0.20-1.5 g are in free form, provided that 5.0-25 %, preferably 6.0-23 %, more preferably 7.0-20 %, still more preferably 8.0-18 %, and most preferably 10-15 % by weight are in free form, and
b) the rest of the equivalents are in esterified form,
- 2.5-15 g, preferably 3.0-12 g, more preferably 4.0-12 g, still more preferably 4.5-12 g, even more preferably 5.0-12 g, and most preferably 6.0-12 g triglyceride fat,
- at least one additional edible ingredient, and
- water.

2. The drink portion according to claim 1, wherein it comprises
- 0.8-8.0 g total plant sterol and plant stanol equivalents, of which equivalents
a) 0.10-1.5 g are in free form, provided that 5.0-25 %, preferably 6.0-23 %, more preferably 7.0-20 %, still more preferably 8.0-18 %, and most preferably 10-15 % by weight are in free form, and
b) the rest of the equivalents are in esterified form, and
- 2.5-12 g triglyceride fat.

3. The drink portion according to claim 1 or 2, wherein the additional edible ingredient(s) comprises protein in an amount of 1.0-35 g, preferably 2.0-33 g, more preferably 3.0-30 g, still more preferably 4.0-28 g even more preferably at least 6.0 g, further more preferably at least 8.0 g, still further more preferably at least 10 g, even further more preferably at least 12 g, and most preferably at least 14 g of the drink portion.

4. A drink with serum LDL cholesterol lowering effect, wherein the drink comprises
- 0.50-10 w/v-%, preferably 0.80-10 w/v-%, more preferably 1.0-10 w/v-%, still more preferably 1.2-10 w/v-%, even more preferably 1.5-10 w/v-% and most preferably 2.0-10 w/v-% total plant sterol and plant stanol equivalents, of which equivalents
a) 5.0-25 %, preferably 6.0-23 %, more preferably 7.0-20 %, still more preferably 8.0-18 %, and most preferably 10-15 % are in free form, and
b) the rest of the equivalents are in esterified form,
- 2.0-12 w/v-%, preferably 3.0-12 w/v-%, more preferably 4.0-12 w/v-%, still more preferably 4.5-12 w/v-%, even more preferably 5.0-12 w/v-%, and most preferably 6.0-12 w/v-% triglyceride fat,
- 0.5-37.5 w/v-% at least one additional edible ingredient, and
- 60-97 w/v-% water.

5. The drink according to claim 4, wherein it comprises
- 1.0-10 w/v-% total plant sterol and plant stanol equivalents, of which equivalents
a) 5.0-25 %, preferably 6.0-23 %, more preferably 7.0-20 %, still more preferably 8.0-18 %, and most preferably 10-15 % are in free form, and
b) the rest of the equivalents are in esterified form, and
- 4.0-12 w/v-%, preferably 4.5-12 w/v-%, more preferably 5.0-12 w/v-%, and most preferably 6.0-12 w/v-% triglyceride fat.

6. The drink according to claim 4 or 5, wherein the additional edible ingredient(s) of the drink comprises protein in an amount of 1.0-12 w/v-%, preferably 3.0-11 w/v-%, more preferably 4.0-11 w/v-%, still more preferably 5.0-11 w/v-%, even more preferably 6.0-11 w/v-%, and most preferably 6.0-9.0 w/v-% of the drink.

7. A pack containing the drink according to any one of claims 4 to 6, or at least one drink portion according to any one of claims 1 to 3.

8. The pack according to claim 7, wherein the pack contains one drink portion.

9. The drink portion according to any one of claims 1 to 3, the drink according to any one of claims 4 to 6, and the pack according to claim 7 or 8, wherein the drink or drink portion has an ensured serum LDL cholesterol lowering effect when used as a snack.

10. The drink portion according to any one of claims 1, 2, 3 or 9, the drink according to any one of claims 4, 5, 6 or 9, and the pack according to any one of claims 7, 8 or 9, wherein the consumer is advised that the drink or drink portion can be used as a snack.

11. The drink portion according to any one of claims 1, 2, 3, 9 or 10, the drink according to any one of claims 4, 5, 6, 9 or 10, and the pack according to any one of claims 7 to 10, wherein the drink or drink portion is a snack.

12. The drink portion according to any one of claims 1, 2, 3, 9, 10 or 11, the drink according to any one of claims 4, 5, 6, 9, 10 or 11, and the pack according to any one of claims 7 to 11, wherein the portion size of the drink or drink portion is 50-500 ml, preferably 50-400 ml, more preferably 65-300 ml, still more preferably 100-300 ml, and most preferably 125-300 ml.

13. The drink portion according to any one of claims 1, 2, 3 or 9 to 12, the drink according to any one of claims 4, 5, 6 or 9 to 12, and the pack according to any one of claims 7 to 12, wherein the energy content of the drink or drink portion is at least 250 kJ/100 ml, more preferably at least 300 kJ/100 ml, still more preferably at least 350 kJ/100 ml, even more preferably at least 400 kJ/100 ml, further more preferably at least 450 kJ/100 ml, still further more preferably at least 500 kJ/100 ml, and most preferably at least 550 kJ/100 ml, and at most 1300 kJ/100 ml, preferably at most 1000 kJ/100 ml, more preferably at most 800 kJ/100 ml, and most preferably at most 700 kJ/100 ml.

14. A powder suitable for making a drink, wherein the powder contains the dry components of the drink portion according to any one of claims 1, 2, 3 or 9-13 per portion of powder, or the dry components at the same ratios as in the drink according to any one of claims 4, 5, 6 or 9-13.

15. A drink portion according to any one of claims 1, 2, 3 or 9 to 13 or a drink according to any one of claims 4, 5, 6 or 9 to 13 or a powder according to claim 14 for use as a medicament for lowering serum LDL cholesterol.

## Patentansprüche

1. Getränkeportion mit Serum-LDL-Cholesterol-senkender Wirkung, wobei die Getränkeportion umfasst:
- 0,8-15 g, vorzugsweise 1,0-15 g, bevorzugter 1,5-12 g, noch bevorzugter 1,8-10 g und höchst bevorzugt 2,0-8,0 g Gesamt-Pflanzensterol und Pflanzenstanol-Äquivalente, von welchen Äquivalenten
a) 0,10-3,0 g, vorzugsweise 0,15-3,0 g, bevorzugter 0,18-2,5 g, noch bevorzugter 0,20-2,0 g und höchst bevorzugt 0,20-1,5 g in freier Form vorliegen, vorausgesetzt, dass 5,0-25 Gew.-%, vorzugsweise 6,0-23 Gew.-%, bevorzugter 7,0-20 Gew.-%, noch bevorzugter 8,0-18 Gew.-% und höchst bevorzugt 10-15 Gew.-% in freier Form vorliegen, und
b) der Rest der Äquivalente in veresterter Form vorliegt,
- 2,5-15 g, vorzugsweise 3,0-12 g, bevorzugter 4,0-12 g, noch bevorzugter 4,5-12 g, sogar noch bevorzugter 5,0-12 g und höchst bevorzugt 6,0-12 g Triglycerid-Fett,
- wenigstens einen zusätzlichen verzehrbaren Inhaltsstoff und
- Wasser.

2. Getränkeportion gemäß Anspruch 1, wobei sie umfasst:
- 0,8-8,0 g Gesamt-Pflanzensterol und Pflanzenstanol-Äquivalente, von welchen Äquivalenten
a) 0,10-1,5 g in freier Form vorliegen, vorausgesetzt, dass 5,0-25 Gew.-%, vorzugsweise 6,0-23 Gew.-%, bevorzugter 7,0-20 Gew.-%, noch bevorzugter 8,0-18 Gew.-% und höchst bevorzugt 10-15 Gew.-% in freier Form vorliegen, und
b) der Rest der Äquivalente in veresterter Form vorliegt und
- 2,5-12 g Triglycerid-Fett.

3. Getränkeportion gemäß Anspruch 1 oder 2, wobei die zusätzlichen verzehrbaren Inhaltsstoffe Protein in einer Menge von 1,0-35 g, vorzugsweise 2,0-33 g, bevorzugter 3,0-30 g, noch bevorzugter 4,0-28 g, sogar noch bevorzugter wenigstens 6,0 g, noch stärker bevorzugt wenigstens 8,0 g, sogar noch stärker bevorzugt wenigstens 10 g, sogar noch weiter bevorzugt wenigstens 12 g und höchst bevorzugt wenigstens 14 g der Getränkeportion umfassen.

4. Getränk mit Serum-LDL-Cholesterol-senkender Wirkung, wobei das Getränk umfasst:
- 0,50-10 w/v-%, vorzugsweise 0,80-10 w/v-%, bevorzugter 1,0-10 w/v-%, noch bevorzugter 1,2-10 w/v-%, sogar noch bevorzugter 1,5-10 w/v-% und höchst bevorzugt 2,0-10 w/v-% Gesamt-Pflanzensterol und Pflanzenstanol-Äquivalente, von welchen Äquivalenten
a) 5,0-25 %, vorzugsweise 6,0-23 %, bevorzugter 7,0-20 %, noch bevorzugter 8,0-18 % und höchst bevorzugt 10-15 % in freier Form vorliegen und
b) der Rest der Äquivalente in veresterter Form vorliegt,
- 2,0-12 w/v-%, vorzugsweise 3,0-12 w/v-%, bevorzugter 4,0-12 w/v-%, noch bevorzugter 4,5-12 w/v-%, sogar noch bevorzugter 5,0-12 w/v-% und höchst bevorzugt 6,0-12 w/v-% Triglycerid-Fett,
- 0,5-37,5 w/v-% an wenigstens einem zusätzlichen verzehrbaren Inhaltsstoff und
- 60-97 w/v-% Wasser.

5. Getränk gemäß Anspruch 4, wobei es umfasst:
- 1,0-10 w/v-% Gesamt-Pflanzensterol und Pflanzenstanol-Äquivalente, von welchen Äquivalenten
a) 5,0-25 %, vorzugsweise 6,0-23 %, bevorzugter 7,0-20 %, noch bevorzugter 8,0-18 % und höchst bevorzugt 10-15 % in freier Form vorliegen und
b) der Rest der Äquivalente in veresterter Form vorliegt und
- 4,0-12 w/v-%, vorzugsweise 4,5-12 w/v-%, bevorzugter 5,0-12 w/v-% und höchst bevorzugt 6,0-12 w/v-% Triglycerid-Fett.

6. Getränk gemäß Anspruch 4 oder 5, wobei die zusätzlichen verzehrbaren Inhaltsstoffe des Getränks Protein in einer Menge von 1,0-12 w/v-%, vorzugsweise 3,0-11 w/v-%, bevorzugter 4,0-11 w/v-%, noch bevorzugter 5,0-11 w/v-%, sogar noch bevorzugter 6,0-11 w/v-% und höchst bevorzugt 6,0-9,0 w/v-% des Getränks umfassen.

7. Packung, enthaltend das Getränk gemäß einem der Ansprüche 4 bis 6 oder wenigstens eine Getränkeportion gemäß einem der Ansprüche 1 bis 3.

8. Packung gemäß Anspruch 7, wobei die Packung eine Getränkeportion enthält.

9. Getränkeportion gemäß einem der Ansprüche 1 bis 3, Getränk gemäß einem der Ansprüche 4 bis 6 und Packung gemäß Anspruch 7 oder 8, wobei das Getränk oder die Getränkeportion bei Verwendung als Snack eine gewährleistete Serum-LDL-Cholesterol-senkende Wirkung aufweist.

10. Getränkeportion gemäß einem der Ansprüche 1, 2, 3 oder 9, Getränk gemäß einem der Ansprüche 4, 5, 6 oder 9 und Packung gemäß einem der Ansprüche 7, 8 oder 9, wobei der Verbraucher darauf hingewiesen wird, dass das Getränk oder die Getränkeportion als Snack verwendet werden kann.

11. Getränkeportion gemäß einem der Ansprüche 1, 2, 3, 9 oder 10, Getränk gemäß einem der Ansprüche 4, 5, 6, 9 oder 10 und Packung gemäß einem der Ansprüche 7 bis 10, wobei das Getränk oder die Getränkeportion ein Snack ist.

12. Getränkeportion gemäß einem der Ansprüche 1, 2, 3, 9, 10 oder 11, Getränk gemäß einem der Ansprüche 4, 5, 6, 9, 10 oder 11 und Packung gemäß einem der Ansprüche 7 bis 11, wobei die Portionsgröße des Getränks oder der Getränkeportion 50-500 ml, vorzugsweise 50-400 ml, bevorzugter 65-300 ml, noch bevorzugter 100-300 ml und höchst bevorzugt 125-300 ml beträgt.

13. Getränkeportion gemäß einem der Ansprüche 1, 2, 3 oder 9 bis 12, Getränk gemäß einem der Ansprüche 4, 5, 6 oder 9 bis 12 und Packung gemäß einem der Ansprüche 7 bis 12, wobei der Energiegehalt des Getränks oder der Getränkeportion wenigstens 250 kJ/100 ml, bevorzugter wenigstens 300 kJ/100 ml, noch bevorzugter wenigstens 350 kJ/100 ml, sogar noch bevorzugter wenigstens 400 kJ/100 ml, stärker bevorzugt wenigstens 450 kJ/100 ml, noch stärker bevorzugt wenigstens 500 kJ/100 ml und höchst bevorzugt wenigstens 550 kJ/100 ml und höchstens 1300 kJ/100 ml, vorzugsweise höchstens 1000 kJ/100 ml, bevorzugter höchstens 800 kJ/100 ml und höchst bevorzugt höchstens 700 kJ/100 ml beträgt.

14. Pulver, geeignet zum Herstellen eines Getränks, wobei das Pulver die Trockenkomponenten der Getränkeportion gemäß einem der Ansprüche 1, 2, 3 oder 9-13 pro Portion an Pulver oder die Trockenkomponenten in den gleichen Verhältnissen wie in dem Getränk gemäß einem der Ansprüche 4, 5, 6 oder 9-13 enthält.

15. Getränkeportion gemäß einem der Ansprüche 1, 2, 3 oder 9 bis 13 oder Getränk gemäß einem der Ansprüche 4, 5, 6 oder 9 bis 13 oder Pulver gemäß Anspruch 14 für die Verwendung als Medikament zur Senkung von Serum-LDL-Cholesterol.

## Revendications

1. Portion de boisson ayant un effet d'abaissement du cholestérol LDL sérique, la portion de boisson comprenant
- 0,8 à 15 g, de préférence 1,0 à 15 g, plus préférablement 1,5 à 12 g, encore plus préférablement 1,8 à 10 g, et de manière préférée entre toutes 2,0 à 8,0 g d'équivalents de stérol de plante et de stanol de plante totaux, équivalents parmi lesquels
a) 0,10 à 3,0 g, de préférence 0,15 à 3,0 g, plus préférablement 0,18 à 2,5 g, encore plus préférablement 0,20 à 2,0 g, et de manière préférée entre toutes 0,20 à 1,5 g sont sous forme libre, à condition que 5,0 à 25 %, de préférence 6,0 à 23 %, plus préférablement 7,0 à 20 %, encore plus préférablement 8,0 à 18 %, et de manière préférée entre toutes 10 à 15 % en poids soient sous forme libre, et
b) le reste des équivalents sont sous forme estérifiée,
- 2,5 à 15 g, de préférence 3,0 à 12 g, plus préférablement 4,0 à 12 g, encore plus préférablement 4,5 à 12 g, encore plus préférablement 5,0 à 12 g, et de manière préférée entre toutes 6,0 à 12 g de lipides triglycérides,
- au moins un ingrédient comestible additionnel, et
- de l'eau.

2. Portion de boisson selon la revendication 1, qui comprend
- 0,8 à 8,0 g d'équivalents de stérol de plante et de stanol de plante totaux, équivalents parmi lesquels
a) 0,10 à 1,5 g sont sous forme libre, à condition que 5,0 à 25 %, de préférence 6,0 à 23 %, plus préférablement 7,0 à 20 %, encore plus préférablement 8,0 à 18 %, et de manière préférée entre toutes 10 à 15 % en poids soient sous forme libre, et
b) le reste des équivalents sont sous forme estérifiée, et
- 2,5 à 12 g de lipides triglycérides.

3. Portion de boisson selon la revendication 1 ou 2, dans laquelle le(s) ingrédient(s) comestible(s) additionnel(s) comprennent une protéine en une quantité de 1,0 à 35 g, de préférence 2,0 à 33 g, plus préférablement 3,0 à 30 g, encore plus préférablement 4,0 à 28 g encore plus préférablement au moins 6,0 g, encore plus préférablement au moins 8,0 g, encore plus préférablement au moins 10 g, encore plus préférablement au moins 12 g, et de manière préférée entre toutes au moins 14 g de la portion de boisson.

4. Boisson ayant un effet d'abaissement du cholestérol LDL sérique, la boisson comprenant
- 0,50 à 10 % m/v, de préférence 0,80 à 10 % m/v, plus préférablement 1,0 à 10 % m/v, encore plus préférablement 1,2 à 10 % m/v, encore plus préférablement 1,5 à 10 % m/v et de manière préférée entre toutes 2,0 à 10 % m/v d'équivalents de stérol de plante et de stanol de plante totaux, équivalents parmi lesquels
a) 5,0 à 25 %, de préférence 6,0 à 23 %, plus préférablement 7,0 à 20 %, encore plus préférablement 8,0 à 18 %, et de manière préférée entre toutes 10 à 15 % sont sous forme libre, et
b) le reste des équivalents sont sous forme estérifiée,
- 2,0 à 12 % m/v, de préférence 3,0 à 12 % m/v, plus préférablement 4,0 à 12 % m/v, encore plus préférablement 4,5 à 12 % m/v, encore plus préférablement 5,0 à 12 % m/v, et de manière préférée entre toutes 6,0 à 12 % m/v de lipides triglycérides,
- 0,5 à 37,5 % m/v d'au moins un ingrédient comestible additionnel, et
- 60 à 97 % m/v d'eau.

5. Boisson selon la revendication 4, **caractérisée en ce qu'**elle comprend
- 1,0 à 10 % m/v d'équivalents de stérol de plante et de stanol de plante totaux, équivalents parmi lesquels
a) 5,0 à 25 %, de préférence 6,0 à 23 %, plus préférablement 7,0 à 20 %, encore plus préférablement 8,0 à 18 %, et de manière préférée entre toutes 10 à 15 % sont sous forme libre, et
b) le reste des équivalents sont sous forme estérifiée, et
- 4,0 à 12 % m/v, de préférence 4,5 à 12 % m/v, plus préférablement 5,0 à 12 % m/v, et de manière préférée entre toutes 6,0 à 12 % m/v de lipides triglycérides.

6. Boisson selon la revendication 4 ou 5, dans laquelle le(s) ingrédient(s) comestible(s) additionnel(s) de la boisson comprennent une protéine en une quantité de 1,0 à 12 % m/v, de préférence 3,0 à 11 % m/v, plus préférablement 4,0 à 11 % m/v, encore plus préférablement 5,0 à 11 % m/v, encore plus préférablement 6,0 à 11 % m/v, et de manière préférée entre toutes 6,0 à 9,0 % m/v de la boisson.

7. Emballage contenant la boisson selon l'une quelconque des revendications 4 à 6, ou au moins une portion de boisson selon l'une quelconque des revendications 1 à 3.

8. Emballage selon la revendication 7, l'emballage contenant une portion de boisson.

9. Portion de boisson selon l'une quelconque des revendications 1 à 3, boisson selon l'une quelconque des revendications 4 à 6, et emballage selon la revendication 7 ou 8, la boisson ou portion de boisson ayant un effet garanti d'abaissement du cholestérol LDL sérique lorsqu'elle est utilisée comme un en-cas.

10. Portion de boisson selon l'une quelconque des revendications 1, 2, 3 ou 9, boisson selon l'une quelconque des revendications 4, 5, 6 ou 9, et emballage selon l'une quelconque des revendications 7, 8 ou 9, le consommateur étant informé que la boisson ou portion de boisson peut être utilisée comme un en-cas.

11. Portion de boisson selon l'une quelconque des revendications 1, 2, 3, 9 ou 10, boisson selon l'une quelconque des revendications 4, 5, 6, 9 ou 10, et emballage selon l'une quelconque des revendications 7 à 10, la boisson ou portion de boisson étant un en-cas.

12. Portion de boisson selon l'une quelconque des revendications 1, 2, 3, 9, 10 ou 11, boisson selon l'une quelconque des revendications 4, 5, 6, 9, 10 ou 11, et emballage selon l'une quelconque des revendications 7 à 11, la taille de portion de la boisson ou portion de boisson étant de 50 à 500 ml, de préférence 50 à 400 ml, plus préférablement 65 à 300 ml, encore plus préférablement 100 à 300 ml, et de manière préférée entre toutes 125 à 300 ml.

13. Portion de boisson selon l'une quelconque des revendications 1, 2, 3 ou 9 à 12, boisson selon l'une quelconque des revendications 4, 5, 6 ou 9 à 12, et emballage selon l'une quelconque des revendications 7 à 12, la teneur énergétique de la boisson ou portion de boisson étant d'au moins 250 kJ/100 ml, plus préférablement au moins 300 kJ/100 ml, encore plus préférablement au moins 350 kJ/100 ml, encore plus préférablement au moins 400 kJ/100 ml, encore plus préférablement au moins 450 kJ/100 ml, encore plus préférablement au moins 500 kJ/100 ml, et de manière préférée entre toutes au moins 550 kJ/100 ml, et au plus 1300 kJ/100 ml, de préférence au plus 1000 kJ/100 ml, plus préférablement au plus 800 kJ/100 ml, et de manière préférée entre toutes au plus 700 kJ/100 ml.

14. Poudre adaptée pour fabriquer une boisson, la poudre contenant les composants secs de la portion de boisson selon l'une quelconque des revendications 1, 2, 3 ou 9 à 13 par portion de poudre, ou les composants secs aux mêmes rapports que dans la boisson selon l'une quelconque des revendications 4, 5, 6 ou 9 à 13.

15. Portion de boisson selon l'une quelconque des revendications 1, 2, 3 ou 9 à 13 ou boisson selon l'une quelconque des revendications 4, 5, 6 ou 9 à 13 ou poudre selon la revendication 14 pour utilisation en tant que médicament pour l'abaissement du cholestérol LDL sérique.
